(19) Europäisches Patentamt
European Patent Office
Office européen des brevets

(11) Numéro de publication: **0 008 269**
**B1**

(12) # FASCICULE DE BREVET EUROPEEN

(45) Date de publication du fascicule du brevet: **01.02.84**

(21) Numéro de dépôt: **79400545.4**

(22) Date de dépôt: **01.08.79**

(51) Int. Cl.³: **C 07 C 147/06,** C 07 C 147/12, C 07 D 295/08, A 61 K 31/10, A 61 K 31/135, A 61 K 31/395

(54) **Nouveaux médicaments contenant, à titre de substance active, des composés de type benzènesulfone, nouveaux composés de ce type et procédé pour leur préparation.**

(30) Priorité: **02.08.78 FR 7822843**

(43) Date de publication de la demande: **20.02.80 Bulletin 80/4**

(45) Mention de la délivrance du brevet: **01.02.84 Bulletin 84/5**

(84) Etats contractants désignés: **BE CH DE GB IT NL**

(56) Documents cités:
**EP - A - 0 007 880**
**DE - C - 926 965**
**DE - C - 949 054**
**DE - C - 952 479**
**FR - A - 783 884**
**FR - A - 1 055 836**
**FR - A - 1 442 292**
**FR - A - 2 116 579**
**FR - A - 2 154 528**
**FR - M - 399**
**GB - A - 544 849**
**US - A - 3 186 904**

**CHEMICAL ABSTRACTS, vol. 75, no. 14, 4 octobre 1971, page 310, ref. 928240y Columbus, Ohio, USA G.E. BERMINGHAM et al: "Ultraviolet absorption spectra of alkyl 0-methoxyphenyl sulfides and oxathiaheterocycles, and of their sulfones"**

**SYNTHESIS 1975, no. 8, August STUTTGART (DE) G.E. VENNSTRA et al. "An improved synthesis of sulfones using tetrabutylammonium sulfinates" pages 519-520**

(73) Titulaire: **CHOAY S.A., 48, Avenue Théophile-Gautier, F-75782 Paris Cédex 16 (FR)**

(72) Inventeur: **Fournier, Jean-Paul, 10, rue Fontenay, F-78000 Versailles (FR)**
Inventeur: **Loiseau, Philippe, 4, Square d'Angiviller, F-78120 Rambouillet (FR)**

(74) Mandataire: **Gutmann, Ernest et al, Cabinet Plasseraud 84, rue d'Amsterdam, F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

# Nouveaux Médicaments contenant, à titre de substance active, des composes de type benzènesulfone, nouveaux composés de ce type et procede pour leur préparation

L'invention est relative à de nouveaux médicaments contenant, à titre de substance active, des composés présentant une structure de base du type benzènesulfone. L'invention est aussi relative aux nouveaux composés servant à l'élaboration de ces nouveaux médicaments. L'invention est enfin relative à un Procédé de préparation de ces composés.

Des sulfones du type $ArSO_2R$ dans lesquelles Ar représente un noyau aromatique et R représente un radical alcoyle, ont été décrites dans:

- les Chemical Abstracts, vol. 75, 1971, p. 310, réf. 92840y qui concerne des composés dans lesquels R peut représenter un groupe méthyle, butyle, isopropyle, terbutyle;
- Synthesis, n° 8, 1975, p. 519–520, qui concerne des composés dans lesquels R peut représenter un groupe alcoyle tel que méthyle et isopropyle;
- le brevet d'invention FR n° 1 442 292 qui concerne des composés dans lesquels R peut représenter un groupe alcoyle contenant de préférence jusqu'à 4 atomes de carbone;
- le brevet US n° 3 186 904 qui concerne des composés dans lesquels R peut représenter un groupe alcoyle;
- le brevet GB n° 544 849 qui concerne des composés dans lesquels R peut représenter un groupe alcoyle.

Des sulfones du type $ArSO_2R$ dans lesquelles R peut représenter le groupe $(CH_2)_n-Cl$ ont été décrites dans:

- le brevet DE n° 952 479 qui concerne des composés de formule $ArSO_2(CH_2)_2Cl$;
- le brevet DE n° 926 965 qui concerne des composés de formule $RSO_2(CH_2)_2Cl$;
- le brevet US n° 3 186 904 qui concerne des composés dans lesquels le radical en alpha du $SO_2$ peut être un radical propyle halogéné ou méthyle halogéné.

Des sulfones du type $ArSO_2R$ dans lesquelles R peut représenter un groupe oxoalkyle, oxoaryle ou ester ont été décrites dans Synthesis, n° 8, 1975, p. 519–520;

Une large catégorie de produits englobant à la fois des sulfones de formule $RSO_2R_1$ et des sulfones de formule $RSOR_1$, dans lesquelles $R_1$ peut représenter un groupe aryle et R représente un radical renfermant un atome d'azote, sont décrites dans le brevet FR n° 783 884.

Mais ces composés exemplifiés dans ce document présentent un substituant en para sur le noyau aromatique et n a pour valeur 2.

Par ailleurs, l'enseignement de ces documents n'indique rien quant à une éventuelle activité pharmacologique des composés qui y sont décrits ou qui y sont suggérés.

Jusqu'à présent, les seules sulfones de type $ArSO_2R$ présentées comme possédant des propriétés pharmacologiques se manifestant par une action sur le système nerveux central, ont un radical R représentant le groupe éthyle.

Il s'agit des parahalogénophényléthylsulfones qui font l'objet du BSM n° 399.

On peut également citer les sulfones du type $ArSO_2CH_2CH_2CH_3$ mentionnées dans le brevet DE n° 949 054 et pour lesquelles il est indiqué, sans aucune précision, qu'elles peuvent être utilisées comme produits pharmaceutiques.

D'autres composés de formule $ArSO_2(CH_2)_nR$ dont le noyau aromatique est substitué en para, dont la valeur de n est 2 et dont la signification de R est un hétérocycle aminé, sont également décrits dans le brevet US n° 3 186 904.

Ces composés sont présentés comme ayant des propriétés biologiques telles qu'antivirales, antifongiques, antidiabétiques.

Des composés de formule $ArSO_2R$ dans lesquels Ar est un aryle non substitué, sont décrits dans la demande de brevet FR n° 2 154 528. Ce document englobe au niveau des formules chimiques, à la fois des composés du type sulfinone et des composés du type sulfone, dont les groupes aryle sont essentiellement dépourvus de substituants.

Ce brevet indique en outre essentiellement en ce qui concerne les sulfinones, que celles-ci ont une activité antalgique.

Le brevet FR n° 2 116 579 concerne également des sulfinones qui ont été présentées comme pouvant être utiles dans le domaine du traitement du dérèglement cardio-vasculaire. Les sulfinones décrites dans ce brevet ainsi que les sulfones correspondantes présentent la formule $ArSO_2R$ dans laquelle Ar est un noyau aromatique et R est soit un radical alkyle ou alcényle, soit un radical hydroxy ayant 2 ou 3 atomes de carbone. Par ailleurs, le noyau aromatique de ces composés comporte toujours un, deux ou trois substituants, ces substituants étant dans le cas d'une monosubstitution un radical alcoxy ou alcoyle de 1 à 3 atomes de carbone.

Les médicaments selon l'invention sont caractérisés en ce qu'ils contiennent, à titre de substance active, au moins un composé du type benzènesulfone répondant à la formule générale:

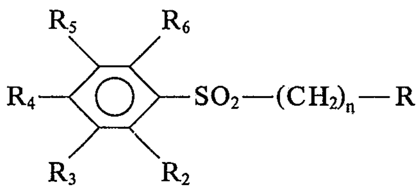

(I)

Dans cette formule,

- n vaut 0, 1, 2 ou 3;
- R est un radical amino ou amino substitué par des groupes alkyle pouvant contenir de 1 à 3 atomes de carbone, ou un hétérocycle azoté;
- $R_2$ à $R_6$ sont indépendamment les uns des autres, un hydrogène, un halogène, $-NO_2$, $-NH_2$, $-CH_3$ ou $-OCH_3$:
  a) sous réserve que l'un au moins des substituants $R_2$ à $R_6$ soit différent de l'hydrogène; et
  b) sous réserve que n soit différent de 2 lorsque $r_4$ est différent de l'hydrogène.

Pour les composés de formule (I) qui présentent un caractère basique, la substance active des médicaments selon l'invention peut aussi consister en un sel de ce composé avec un acide pharmaceutiquement acceptable.

Dans une forme préférée, les médicaments selon l'invention contiennent, à titre de substance active, au moins un composé de formule (I) dans laquelle $R_2$ est $-OCH_3$.

Dans une autre forme préférée, les médicaments selon l'invention contiennent, à titre de substance active, au moins un composé de formule suivante:

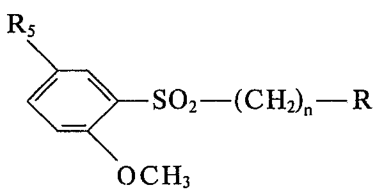

dans laquelle $R_5$ représente un atome d'hydrogène, de brome, notamment de chlore.

Dans une autre forme préférée, les médicaments selon l'invention contiennent, à titre de substance active, au moins un composé de formule (I) dans laquelle R est un groupe morpholino, pyrrolidino, pipéridino.

Dans une autre forme préférée, les substances actives pour les médicaments selon l'invention sont celles de formule (I) dans laquelle n est égal à 2 ou 3, $R_5$ est un atome de chlore ou d'hydrogène, et R est un groupe pipéridino, morpholino ou pyrrolidino, ou encore un sel de ces composés avec un acide pharmaceutiquement acceptable.

Pour former les médicaments selon l'invention, des substances actives préférées sont les composés suivants:

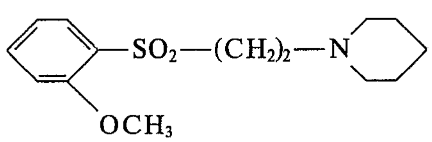

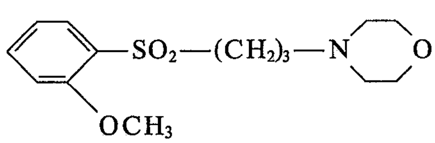

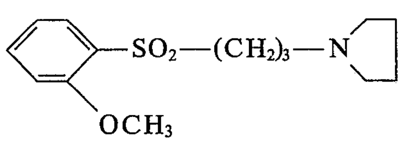

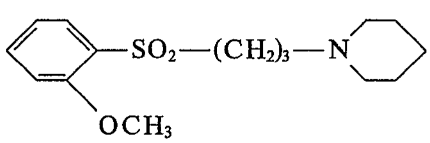

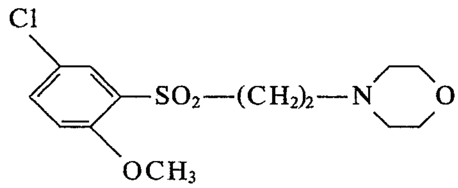

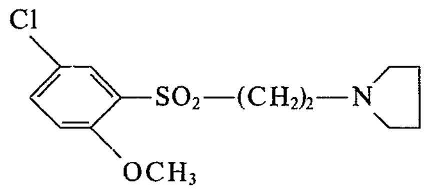

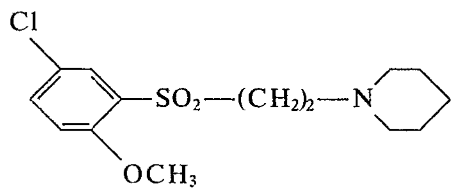

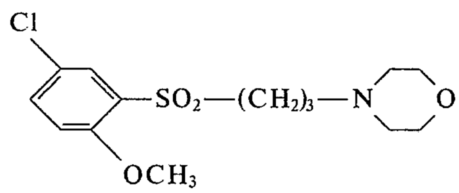

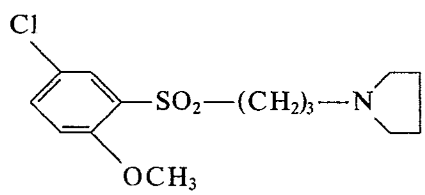

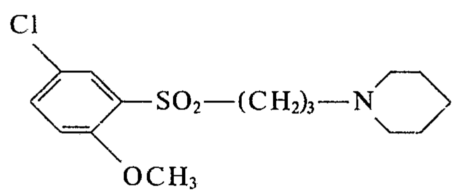

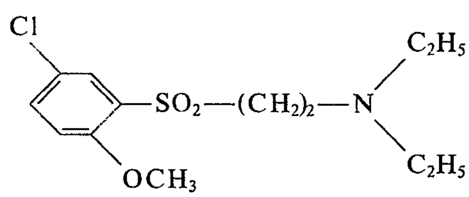

Les médicaments selon l'invention présentent un ensemble de propriétés pharmacologiques et thérapeutiques qui en font des produits de grande valeur.

Les propriétés les plus remarquables se manifestent dans le domaine des troubles de la lipidémie. Ils permettent notamment d'abaisser le taux de lipides circulants chez un sujet présentant une hyperlipidémie. Ces médicaments sont en conséquence particulièrement utiles pour le traitement de troubles de la lipémie tels que l'hypercholestérolémie ou l'hypertriglycéridémie qui précèdent ou accompagnent notamment les maladies athéromateuses.

Les médicaments selon l'invention peuvent influer également sur d'autres facteurs sériques; ils peuvent notamment présenter des activités modifiant la glycémie ou des facteurs plasmatiques de la coagulation.

Les médicaments selon l'invention peuvent aussi agir sur les troubles dans lesquels intervient le

système nerveux central. Ils peuvent ainsi manifester des propriétés tranquillisantes, anticonvulsivantes, antiémétiques, antiulcérogènes, etc.

Ils peuvent encore influer sur les mécanismes de la mitose.

Aux doses actives, en particulier lorsqu'ils sont utilisés pour leurs propriétés normolipémiantes, les médicaments selon l'invention sont dépourvus de toxicité. Le rapport dose active/dose toxique peut être comparé favorablement à celui de substances connues présentant des propriétés de même nature.

En plus des substances actives constituées par les composés de formule (I), les médicaments selon l'invention peuvent contenir d'autres substances actives compatibles avec les premières.

Dans ces médicaments selon l'invention, les substances actives sont associées, dans la mesure où cela est nécessaire, avec des excipients et adjuvants traditionnels destinés à faciliter et améliorer leur utilisation, leur conservation, etc. En particulier, les substances actives sont associées aux excipients solides ou liquides facilitant leur administration en fonction de la voie d'introduction.

Compte tenu de leurs activités et des traitements pour lesquels ils sont utilisés, les médicaments selon l'invention sont avantageusement administrés par voie parentérale ou par voie orale. Ils peuvent, à cet effet, être présentés sous des formes très variées: comprimés, dragées, capsules, gélules, poudres, solutions, suspensions, sirops, suppositoires ...

Ils peuvent aussi, en association avec des composés lipidiques, conduire à la formation de présentations pharmaceutiques du type liposome.

Les composés nouveaux selon l'invention, utiles pour la préparation de médicaments tels que décrits précédemment, répondent à la formule générale (I) dans laquelle

- n vaut 0, 1, 2 ou 3;
- R est un radical amino ou amino substitué par des groupes alkyle pouvant contenir de 1 à 3 atomes de carbone, ou un hétérocycle azoté;
- $R_2$ à $R_6$ sont indépendamment les uns des autres, un hydrogène, un halogène, $-NO_2$, $-NH_2$, $-CH_3$ ou $-OCH_3$:
  a) sous réserve que l'un au moins des substituants $R_2$ à $R_6$ soit différent de l'hydrogène; et
  b) sous réserve que n soit différent de 2 lorsque $R_4$ est différent de l'hydrogène.

Dans un groupe préféré de composés selon l'invention, $R_2$ est $-OCH_3$.

Un autre groupe préféré de composés selon l'invention, répond à la formule suivante:

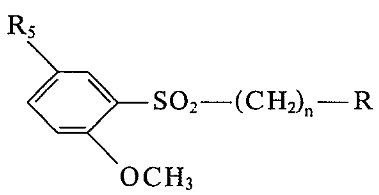

dans laquelle $R_5$ représente un atome d'hydrogène, de brome, notamment de chlore.

Dans un autre groupe préféré de composés selon l'invention, R est un groupe pyrrolidino, pipéridino ou morpholino.

Dans un autre groupe préféré de composés selon l'invention, n est égal à 2 ou 3, $R_5$ est un atome de chlore ou d'hydrogène, et R est un groupe pipéridino, morpholino ou pyrrolidino.

Des composés particulièrement préférés selon l'invention sont ceux de formules:

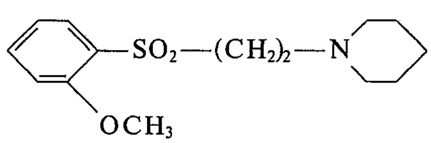

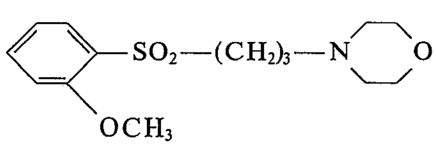

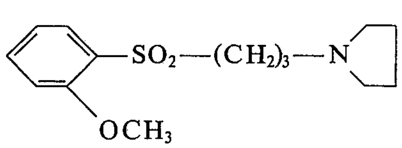

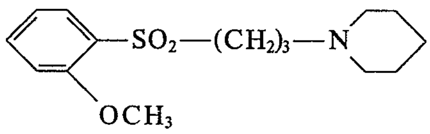

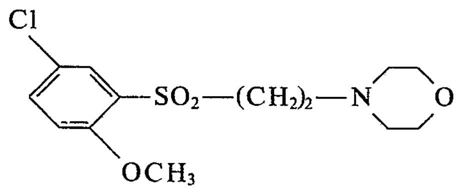

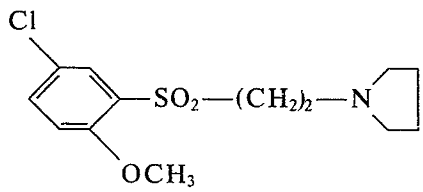

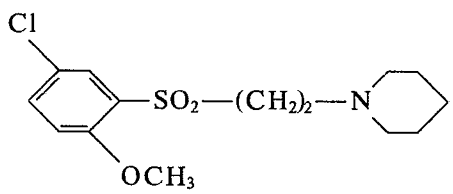

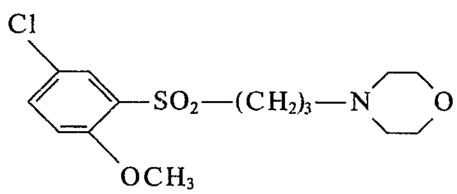

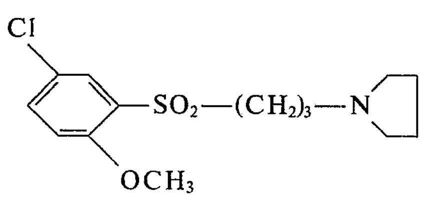

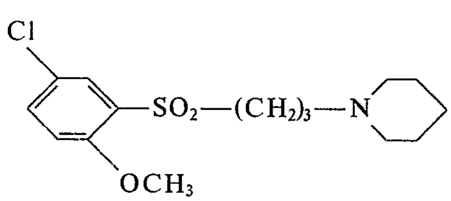

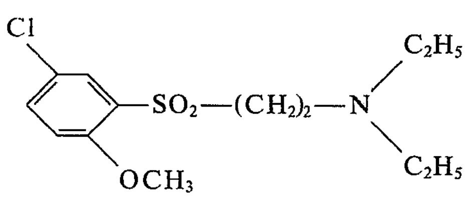

Pour obtenir les composés de formule générale (I) dans lesquels R est un groupe amino éventuellement substitué ou un hétérocycle azoté, on peut partir d'un composé (II) approprié dans lequel R est un atome de chlore, et en effectuant une condensation avec le composé aminé ou à azote cyclique correspondant suivant le schéma:

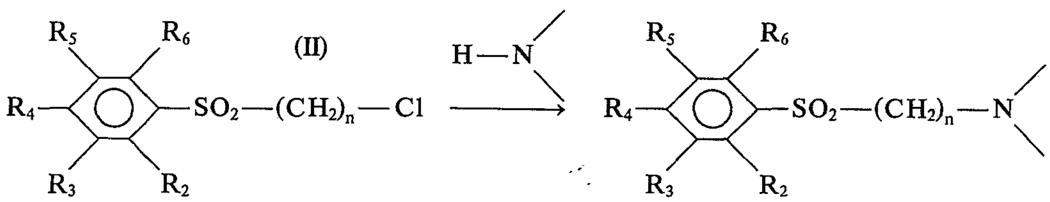

Un mode de préparation du composé de formule (II) consiste à partir d'un benzènethiol approprié et à le faire réagir avec un composé halogéné en présence de sodium suivant le schéma:

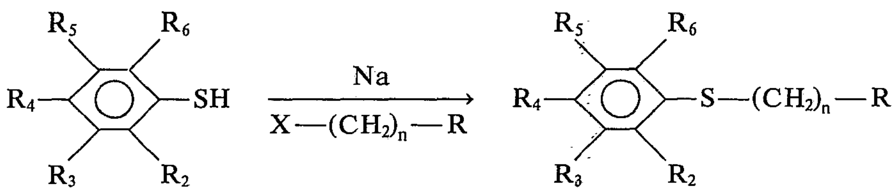


Dans ces formules, n, $R_2$, $R_3$, $R_4$, $R_5$, $R_6$ ont les significations indiquées précédemment, et R représente un atome de chlore.

Le sulfure obtenu dans le premier temps est transformé suivant un mode connu en sulfone, par exemple par oxydation àe l'eau oxygénée en milieu acétique.

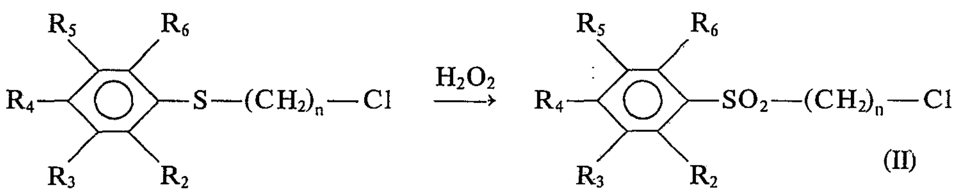


(II)

Les exemples suivants illustrent des modes de préparation de composés selon l'invention.

## Exemple 1

Mode de formation d'un thiol lorsque celui-ci n'est pas disponible

a) Chloro-5 méthoxy-2 benzènethiol

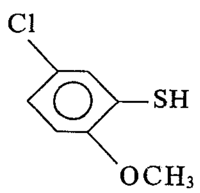

On met en solution 0,25 mole (39,4 g) d'amino-2 chloro-4 anisole dans 40 ml d'acide chlorhydrique (d = 1,18). On diazote l'amine à 0°C par addition de 17,2 g de nitrite de sodium en solution dans 110 ml d'eau. La réaction terminée, on tamponne le milieu par la quantité nécessaire d'acétate de sodium.

On verse lentement (réaction exothermique) le sel de diazonium sur une solution de 0,25 mole (40 g) de dithiocarbonate de potassium et d'éthyl-xanthate de potassium dans 250 ml d'eau portée à 75—80°C. On assure un reflux pendant 1 heure. Après refroidissement, on extrait l'huile obtenue par trois fois 100 ml d'éther éthylique. On sépare la phase organique, on la sèche sur sulfate de sodium et on l'évapore sous pression réduite. On ajoute l'huile résiduelle à une solution de 40 g d'hydroxyde de sodium dans 250 ml de méthanol et 40 ml d'eau. On porte le Mélange à reflux sous azote 2 heures, puis on le refroidit et on l'acidifie par de l'acide chlorhydrique. On obtient une huile que l'on extrait par trois fois 100 ml d'éther éthylique; on sèche la phase éthérée sur sulfate de sodium et on l'évapore sous pression réduite.

Rendement 61% — P. F. 42°C.

b) Méthoxy-2 benzènethiol: même protocole (formule II avec

$R_2 = OCH_3$
$R_3 = R_4 = H$)

Rendement 31% — P.E. 82—84°C à 0,4—0,6 mm de Hg

## Exemple 2

### Préparation de sulfures

a) Sulfure de chloro-2 éthyle et de chloro-5′ méthoxy-2′ phényle

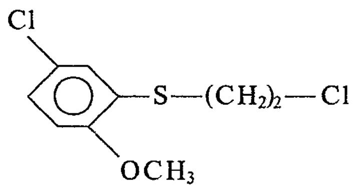

Dans un ballon à trois cols de 250 ml, muni d'un systéme d'agitation, d'un thermomètre et d'une ampoule à brome, on introduit 100 ml de Méthanol que l'on refroidit vers 0°C; on ajoute par petites fractions 5 g de sodium; lorsque celui-ci est complètement dissous, on introduit 0,075 mole (13 g) de chloro-5 méthoxy-2 benzènethiol obtenu comme indiqué à l'exemple 1, puis goutte à goutte 0,09 mole de bromo-1 chloro-2 éthane (13 g) en opérant sous atmosphère d'azote durant toute l'opération. On ramène le mélange à température ambiante, puis on le chauffe vers 60°C au bain-marie; le milieu réactionnel se trouble, on laisse en contact 30 minutes et on verse sur de la glace pilée. On extrait le sulfure par trois fois 50 ml de toluène; la phase organique est séparée, séchée sur sulfate de sodium et évaporée sous pression réduite.

b) Sulfure de chloro-3 propyle et de chloro-5′ méthoxy-2′ phényle

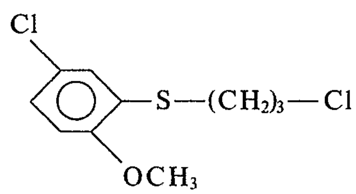

On opère comme en a) en utilisant cette fois le bromo-1 chloro-3 propane.

c) Sulfure de chloro-2 éthyl et de méthoxy-2′ phényle

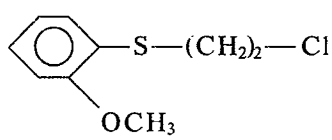

On opère de la même façon en utilisant cette fois le méthoxy-2 benzènethiol et le bromo-1 chloro-2 éthane.

d) Sulfure de chloro-3-propyl et de méthoxy-2′ phényle.

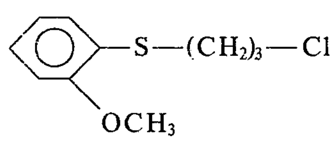

La même opération est répétée avec le méthoxy-2 benzènethiol et le bromo-1 chloro-3 propane.

## Exemple 3

### Formation de chloro-alkyl phényl sulfones

On verse un sulfure tel qu'obtenu précédemment à l'exemple 2 dans une solution contenant 30 ml d'eau oxygénée à 110 volumes et 80 ml d'acide acétique pur. On porte le mélange à 80°C au bain-marie 2 heures, puis on le verse sur de la glace pilée. La sulfone précipite. On la cristallise dans un mélange éthanol-eau.

Les sulfones suivantes ont été préparées. Les rendements sont calculés à partir du benzènethiol.

a) Chloro-2 éthyl (chloro-5 méthoxy-2) phényl sulfone

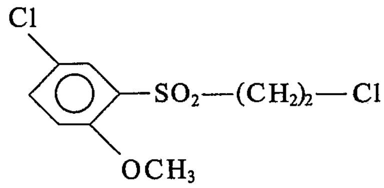

Rendement 65% – P.F. 79°C.

b) Chloro-3 propyl (chloro-5 méthoxy-2) phényl sulfone

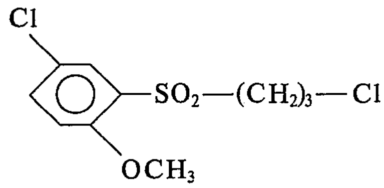

Rendement 69% – P.F. 110°C.

c) Chloro-2 éthyl (méthoxy-2) phényl sulfone

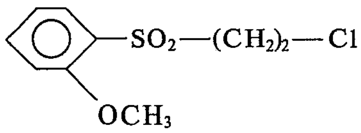

Rendement 40% – P.F. 32°C.

d) Chloro-3 propyl (méthoxy-2) phényl sulfone

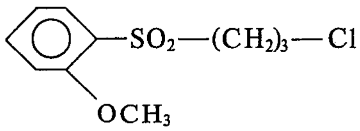

Rendement 54% – P.F. 66°C.

## Exemple 4

Préparation d'un compose selon l'invention contenant un hétérocycle azoté

Pipéridino-2 éthyl (chloro-5 méthoxy-2) phényl sulfone

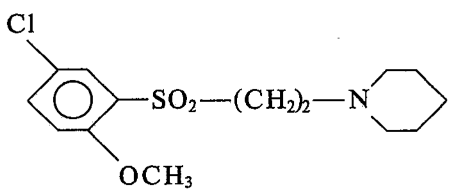

On dissout 0,01 mole (2,69 g) de chloro-2 éthyl (chloro-5 méthoxy-2) phényl sulfone dans 40 ml d'isopropanol, on ajoute 0,03 mole de pipéridine et on porte à reflux 12 heures. On évapore la solution sous pression réduite, puis on reprend le résidu par trois fois 10 ml de chloroforme après dilution du milieu par 50 ml d'eau. La sulfone base extraite est cristallisée dans un mélange éthanol-eau.

On passe au sel correspondant par addition de la quantité stoechiométrique d'acide.

## Exemple 5 à 27

On a préparé une série d'alkyl phényl sulfones selon le procédé décrit à l'exemple 4.

On a indiqué au tableau suivant la nature de ces composés par l'indication des substituants de la formule (I). On a donné également la forme du composé, à savoir s'il est sous forme de base ou de sel d'acide, le poids moléculaire, le point de fusion et le rendement. Les substituants $R_3$ et $R_6$ sont l'hydrogène dans tous les cas.

| Ex. n° | n | R | $R_2$ | $R_4$ | $R_5$ | Forme | P.M. | P.F. °C | Rendement % |
|---|---|---|---|---|---|---|---|---|---|
| 5 | 2 | Cl | $OCH_3$ | H | H | | 234,7 | 32 | 40 |
| 6 | 3 | Cl | $OCH_3$ | H | H | | 248,7 | 66 | 54 |
| 7 | 2 | Cl | $OCH_3$ | H | Cl | | 269,1 | 79 | 65 |
| 8 | 3 | Cl | $OCH_3$ | H | Cl | | 283,1 | 110 | 69 |
| 9 | 2 | 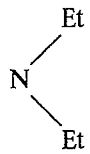 | $OCH_3$ | H | Cl | ,HCl | 342,3 | 130 | 52 |
| 18 | 2 | 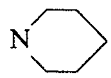 | $OCH_3$ | H | H | picrate | 512,5 | 162 | 32 |
| 19 | 3 | 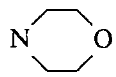 | $OCH_3$ | H | H | picrate | 528,5 | 149 | 41 |
| 20 | 3 | 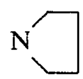 | $OCH_3$ | H | H | picrate | 512,5 | 144 | 39 |
| 21 | 3 | 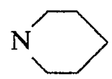 | $OCH_3$ | H | H | picrate | 526,5 | 138 | 44 |
| 22 | 2 | 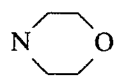 | $OCH_3$ | H | Cl | base | 319,8 | 90 | 47 |
| | | | | | | ,HCl | 356,3 | 192 | |
| 23 | 2 | 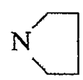 | $OCH_3$ | H | Cl | base | 303,8 | 73 | 62 |
| | | | | | | ,HCl | 340,3 | 197 | |
| 24 | 2 | 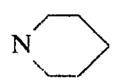 | $OCH_3$ | H | Cl | base | 317,8 | 110 | 53 |
| 25 | 3 | 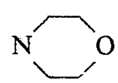 | $OCH_3$ | H | Cl | ,HCl | 370,3 | 237 | 65 |
| 26 | 3 | 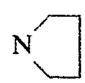 | $OCH_3$ | H | Cl | ,HCl | 354,3 | 172 | 57 |
| 27 | 3 | 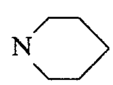 | $OCH_3$ | H | Cl | ,$CH_3SO_3H$ | 427,9 | 131 | 46 |

Pour tous les produits synthétisés, on s'est assuré par des techniques de micro-analyse, par spectres RMN et IR, de ce que le produit obtenu correspond bien au produit visé.

A titre d'exemples, différents essais pour la mise en évidence des propriétés des médicaments selon l'invention sont rapportés ci-après.

a) Toxicité

La détermination est faite sur des souris Swiss de 26 à 30 g. Le produit est administré, en solution dans du soluté isotonique, par voie intraveineuse ou par voie intrapéritonéale (la solution dans ce cas contient en outre 3% de gomme arabique). Le volume administré est de 0,5 ml par 20 g de poids. Les animaux sont mis en observation pendant 8 jours et la dose létale 50 est calculée suivant la méthode de Behrens et Karber (Arch. Exp. Path. Pharmacol., 1935, 177, 37 a).

Les essais effectués ont montré que la dose létale 50 des substances actives était très élevée. Ainsi, elle s'établit, notamment pour les substances des exemples 22, 24, 26 et 27, respectivement à:

| | |
|---|---|
| ex. 22 $DL_{50}$ | |
| intraveineux | 212,5 mg/kg |
| intrapéritonéal | 537,5 mg/kg |
| ex. 24 $DL_{50}$ | |
| intrapéritonéal | 430 mg/kg |
| ex. 26 $DL_{50}$ | |
| intraveineux | 105 mg/kg |
| intrapéritonéal | 390 mg/kg |
| ex. 27 $DL_{50}$ | |
| intraveineux | 83 mg/kg |

b) Activité normolipémiante et/ou hypoglycémiante

Les essais sont réalisés sur des groupes de 5 rats Wistar mâles (150 à 175 g). Les produits testés sont administrés soit par voie intrapéritonéale, à raison de 100 mg/kg dans du soluté isotonique, soit par voie orale, en solution aqueuse à 3% de gomme adragante (1 ml/100 g de poids corporel) en quantité correspondant à 500 mg/kg.

Pour ces essais, on provoque une hyperlipidémie chez les animaux au moyen du produit commercialisé par la Société ROHM et HAAS sous le nom de Triton W. R. Chez le rat à jeun, l'injection intraveineuse de Triton entraîne une hyperlipidémie se manifestant de manière particulièrement nette pour les triglycérides et de façon moindre pour les lipides totaux.

Cette hyperlipidémie est très nettement abaissée par l'action des substances actives selon l'invention.

Le protocole de l'essai est le suivant. Les rats sont mis à jeûner pendant 18 heures. Le Triton W. R. est administré par voie intraveineuse en solution aqueuse à 10% à raison de 200 mg/kg.

Simultanément, les rats reçoivent les produits à tester de la façon indiquée ci-dessus. Six heures plus tard, on effectue les dosages de façon traditionnelle sur des échantillons sanguins.

La comparaison des résultats obtenus avec les témoins non traités ou ceux ayant reçu le Triton seul montre l'efficacité des substances actives pour le contrôle de la lipémie.

e) Activité anticonsulsivante

Les essais sont réalisés sur des lots de 10 souris Swiss (26—30 g). Les substances sont administrées en solution ou suspension dans du soluté isotonique à raison de 50 mg/kg (50 mg dans 25 ml de soluté) par voie intrapéritonéale.

Pour déclencher les convulsions cardiaques, on utilise le pentétrazol administré, à raison de 100 mg/kg également par voie intrapéritonéale, 30 minutes après l'injection de la substance testée.

Le temps d'apparition des convulsions cloniques, toniques, puis de la mort est mesuré et comparé avec ceux d'un lot de souris témoins.

Pour les substances des exemples 24 et 27, l'accroissement du temps correspondant à ces différents symptômes par rapport aux témoins est respectivement:

| | Exemple 24 | Exemple 27 |
|---|---|---|
| convulsions cloniques | 35,5% | 14,8% |
| convulsions toniques | 33 | 60,2 |
| mortalité | 31,4 | 70,9 |

d) Activité antifibrillante

Pour ses essais, on utilise comme précédemment des lots de 10 souris auxquelles sont administrés, par voie intrapéritonéale, 0,5 ml d'une solution ou suspension de substance étudiée dans du soluté isotonique.

Les souris sont traitées suivant une méthode voisine de celle décrite par B. Vargaftig et J. L. Coignet (Eur. J. Pharmacol., 1969, 6, 49). Selon cette méthode, les souris sont soumises à une atmosphère chargée d'une quantité déterminée de chloroforme. Lorsque les mouvements respiratoires cessent, le thorax est ouvert et le coeur examiné pour l'observation des tremblements superficiels très rapides. La fibrillation est positive lorsque ces mouvements sont observés pendant plus de 5 secondes.

La substance étudiée est administrée à raison de 100 mg/kg 15 minutes avant l'exposition au chloroforme.

La protection observée pour les substances des exemples 22 et 27 est d'environ 30%.

e) Activité antiémétique

Les essais sont effectués sur des chiens Beagle adultes de 8 à 12 kg, qui sont mis à jeun pendant 24 heures.

Chaque animal est son propre témoin, un essai sans protection étant réalisé 8 jours avant l'essai proprement dit en présence de la substance étudiée.

Les substances étudiées sont administrées dans du soluté isotonique à raison de 1 mg/kg et par voie intramusculaire. 30 minutes après, on injecte l'apomorphine par voie sous-cutanée, à raison de 0,1 mg/kg, et l'on dénombre les vomissements dans la demi-heure suivante.

Dans ces conditions, on a observé, pour les substances des exemples 22, 24, 26 et 27, une réduction du nombre de vomissements qui est respectivement de 50,8%, 28,2%, 44,2%, 47,6%.

f) Activité antiulcérogène (ulcère de contrainte)

Elle est déterminée sur les ulcères provoqués par immobilisation chez le rat. La méthode de mesure est celle décrite par G. Narcisse (Thérapie, 1972, 27, 705). La substance est administrée par voie intrapéritonéale, à raison de 10 mg/kg.

Par comparaison avec des témoins, le pourcentage de protection, dans le cas du produit de l'exemple 22, est de 61,3%.

**Revendications**

1. Composé nouveau utile pour la préparation de médicaments, caractérisé en ce qu'il répond à la formule générale suivante:

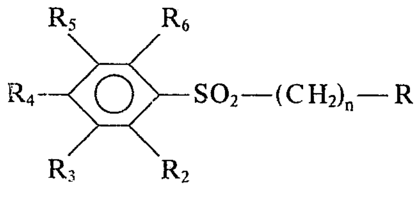

dans laquelle:

- n vaut 0, 1, 2 ou 3;
- R est un radical amino ou amino substitué par des groupes alkyle pouvant contenir de 1 à 3 atomes de carbone, ou un hétérocycle azoté;
- $R_2$ à $R_6$ sont indépendamment les uns des autres, un hydrogène, un halogène, $-NO_2$, $-NH_2$, $-CH_3$ ou $-OCH_3$:
  - a) sous réserve que l'un au moins des substituants $R_2$ à $R_6$ soit différent de l'hydrogène; et
  - b) sous réserve que n soit différent de 2 lorsque $R_4$ est différent de l'hydrogène;

ou pour les composés à caractère basique, les sels correspondants d'acides pharmaceutiquement acceptables.

2. Composé selon la revendication 1, caractérisé en ce que $R_2$ représente le groupe $OCH_3$.

3. Composé selon la revendication 2, caractérisé en ce qu'il répond à la formule suivante:

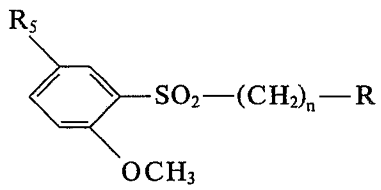

dans laquelle $R_5$ représente un atome d'hydrogène, de brome, notamment de chlore.

4. Composé selon l'une quelconque des revendications 1 à 3, caractérisé en ce que R est un groupe morpholino, pyrrolidino, pipéridino.

5. Composé selon l'une quelconque des revendications 1 à 4, caractérisé en ce que:

- n est égal à 2 ou 3,
- $R_5$ est un atome de chlore ou d'hydrogène,
- R est un groupe pipéridino, morpholino ou pyrrolidino.

6. Composé selon la revendication 1, caractérisé en ce qu'il présente l'une des formules suivantes:

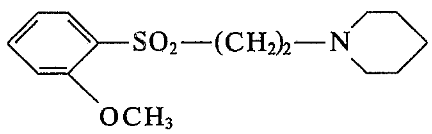

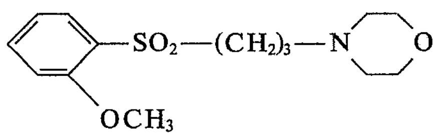

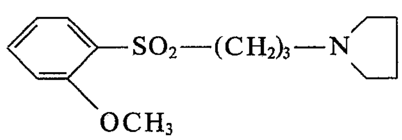

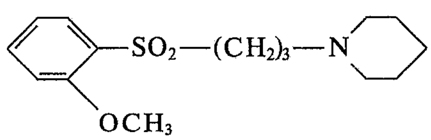

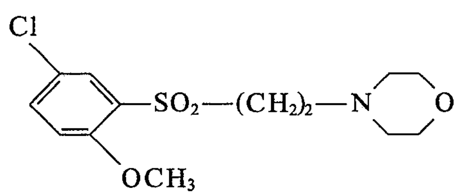

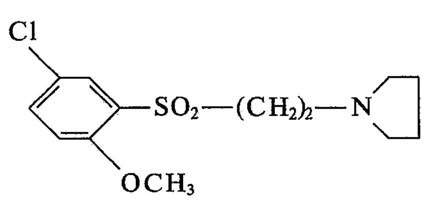

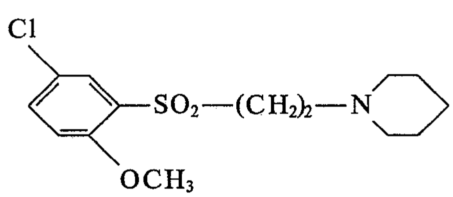

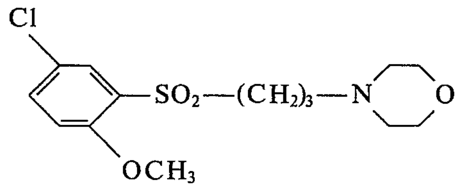

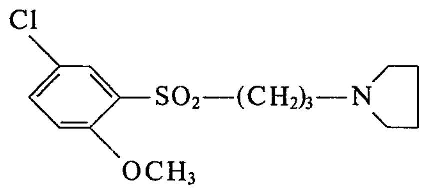

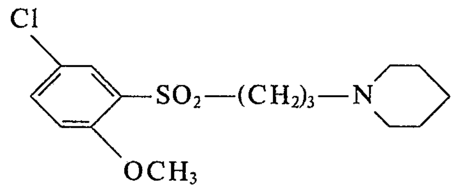

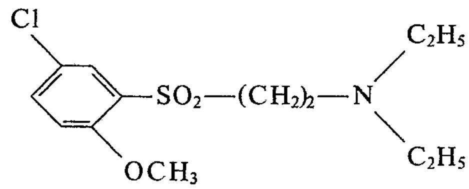

7. Médicament caractérisé en ce qu'il contient, à titre de substance active, au moins un composé de type benzènesulfone selon l'une quelconque des revendications 1 à 6.

8. Procédé de préparation de composés de formule générale (I) dans lesquels R est un groupe amino éventuellement substitué ou un hétérocycle azoté, dans lequel on part d'un composé (II) approprié dans lequel R est un atome de chlore, et on effectue une condensation avec le composé aminé ou à azote cyclique correspondant suivant le schéma:

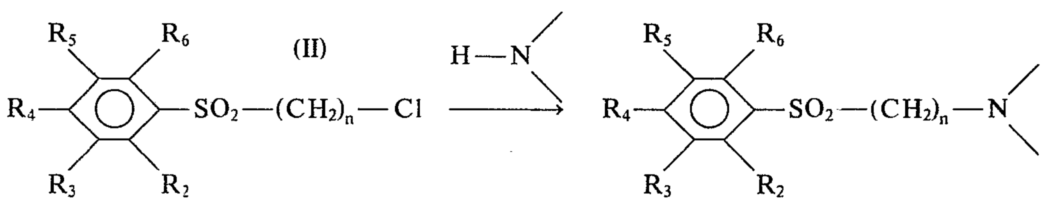
(II)

**Patentansprüche**

1. Verbindungen, die geeignet sind zur Herstellung von Medikamenten, dadurch gekennzeichnet, daß sie die folgende allgemeine Formel aufweisen:

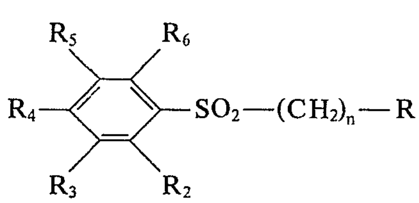
(I)

worin:

n 0, 1, 2 oder 3 bedeutet;

R ein Aminorest ist oder ein Aminorest, der durch Alkylgruppen mit 1 bis 3 Kohlenstoffatomen substituiert ist oder ein Stickstoffheterocyklus ist;

$R_2$ bis $R_6$ unabhängig voneinander Wasserstoff, Halogen, $-NO_2$, $-NH_2$, $-CH_3$ oder $-OCH_3$ bedeuten:

a) mit dem Vorbehalt, daß mindestens einer der Substituenten $R_2$ bis $R_6$ verschieden von Wasserstoff sein muß und

b) unter dem Vorbehalt, daß n verschieden von 2 ist, wenn $R_4$ verschieden von Wasserstoff ist;

oder die pharmazeutisch annehmbaren korrespondierenden Salze bei basischen Verbindungen.

2. Verbindung nach Anspruch 1, dadurch gekennzeichnet, daß $R_2$ die Gruppe $OCH_3$ bedeutet.

3. Verbindung nach Anspruch 2, dadurch gekennzeichnet, daß sie die allgemeine Formel:

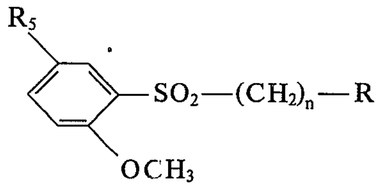

hat, in welcher $R_5$ Wasserstoff, Brom und insbesondere Chlor bedeutet.

4. Verbindung nach einem der Ansprüche 1 bis 3, dadurch gekennzeichnet, daß R ein Morpholino-, Pyrrolidino- oder Piperidinorest ist.

5. Verbindung nach einem der Ansprüche 1 bis 4, dadurch gekennzeichnet, daß n 2 oder 3 ist, $R_5$ ein Chlor- oder Wasserstoffatom ist, R eine Piperidino-, Morpholino- oder Pyrrolidinogruppe ist.

6. Verbindung nach Anspruch 1, dadurch gekennzeichnet, daß sie durch eine der folgenden Formeln dargestellt wird:

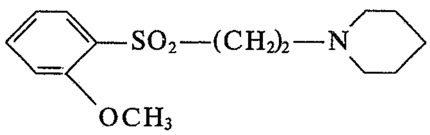

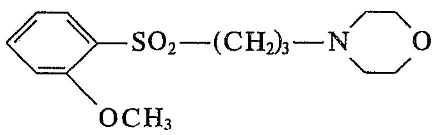

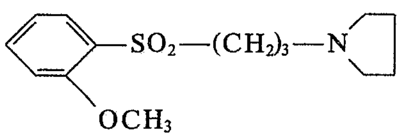

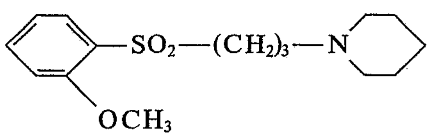

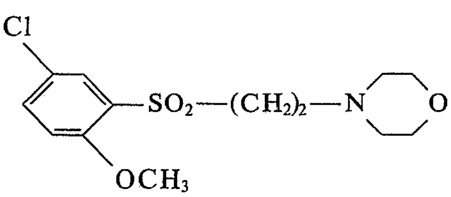

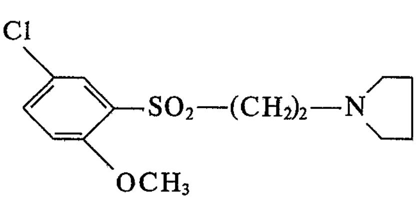

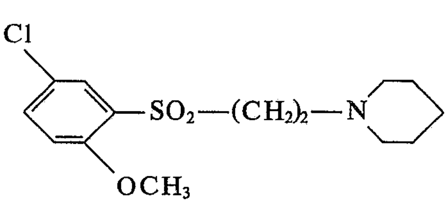

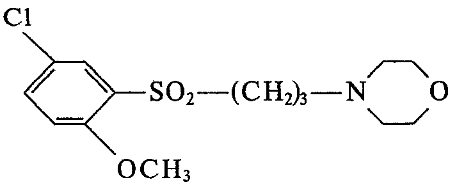

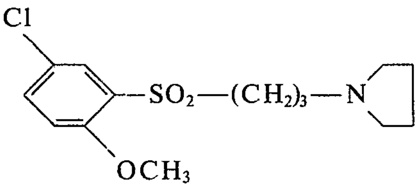

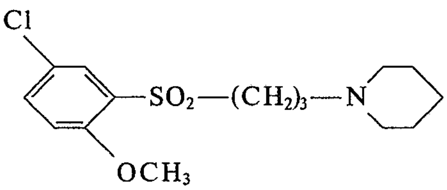

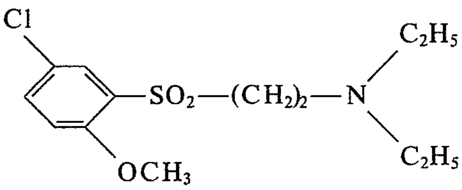

7. Medikament, dadurch gekennzeichnet, daß es als aktive Substanz mindestens eine Verbindung des Benzolsulfontyps gemäß einem der Ansprüche 1 bis 6 enthält.

8. Verfahren zur Herstellung der Verbindungen der allgemeinen Formel (I), in welcher R eine Aminogruppe ist, die gegebenenfalls substituiert ist oder ein Stickstoffheterocyclus ist, wobei man ausgeht von einer Verbindung mit der allgemeinen Formel (II), in welcher R ein Chloratom bedeutet, und eine Kondensation mit einer Aminverbindung oder einer cyclischen Stickstoffverbindung gemäß dem folgenden Schema durchführt:

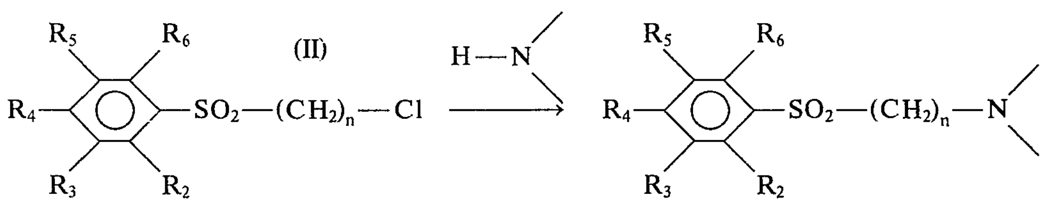
(II)

**Claims**

1. New compound useful for preparing drugs, characterized by the fact that they have the following formula:

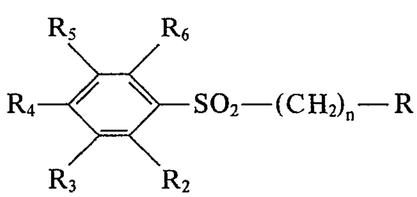
(I)

in which:

- n is 0, 1, 2 or 3;
- R is an amino radical or an amino radical substituted by alkyl groups which can contain from 1 to 3 carbon atoms, or a nitrogen containing heterocycle;
- $R_2$ to $R_6$ are independently from each other a hydrogen, an halogen, $-NO_2$, $-NH_2$, $-CH_3$ or $-OCH_3$:
  a) provided that one at least of the substituents $R_2$ to $R_6$ is different from hydrogen; and
  b) provided that n is different from 2 when $R_4$ is different from hydrogen;

or for the compounds with basic character, the pharmaceutically acceptable corresponding salts of acids.

2. Compound according to claim 1, characterized by the fact that $R_2$ represents the $OCH_3$ group.

3. Compound according to claim 2, characterized by the fact that it has the following formula:

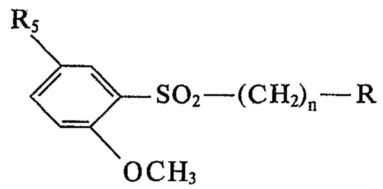

in which $R_5$ represents a hydrogen atom, a bromine atom or preferably a chlorine atom.

4. Compound according to anyone of claims 1 to 3, characterized by the fact that R is a morpholino, pyrrolidino, piperidino group.

5. Compound according to anyone of claims 1 to 4, characterized by the fact that:

- n is 2 or 3;
- $R_5$ is a chlorine or hydrogen atom;
- R is a piperidino, morpholino or pyrrolidino group.

6. Compound according to claim 1, characterized by the fact that it has one of the following formulae:

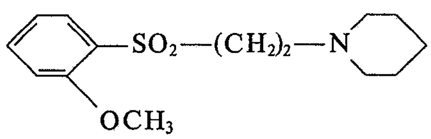

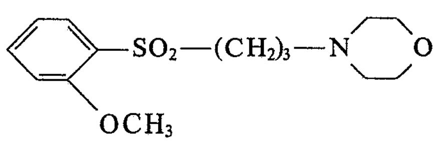

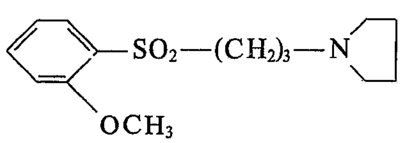

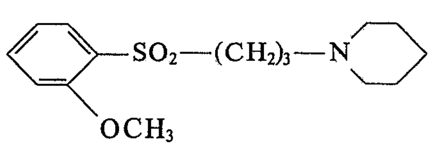

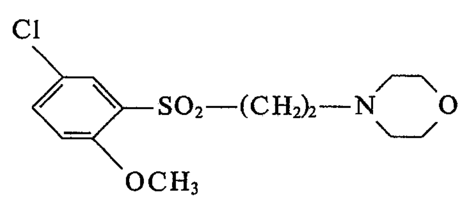

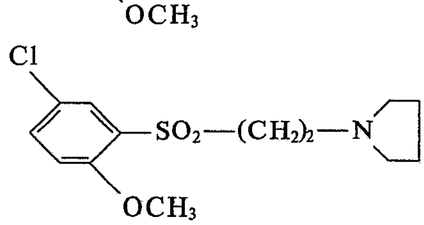

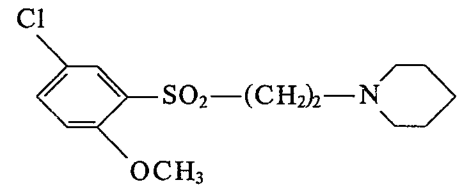

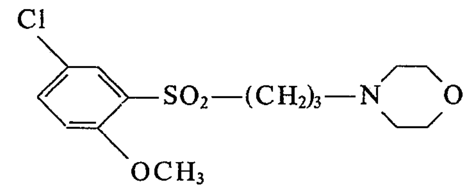

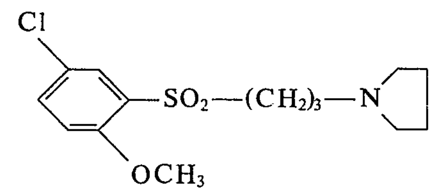

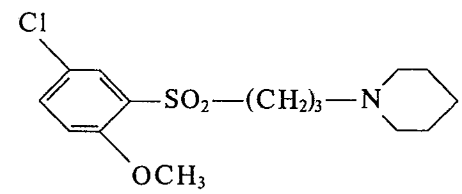

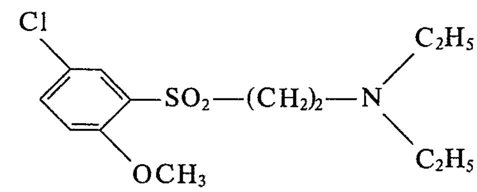

7. Drug characterized by the fact that it contains, as active substance, at least one compound of the benzenesulfone type according to anyone of claims 1 to 6.

8. Process for preparing compounds of general formula (I), in which R is an amino group possibly substituted or a nitrogen containing heterocycle, in which the starting material is an appropriate compound (II) in which R is a chlorine atom and is reacted with the corresponding aminated compound or with the cyclic nitrogen compound according to the following reaction: